# EUROPEAN PATENT APPLICATION

(11) **EP 0 992 218 A1**
(43) Date of publication of application: **12.04.2000**
(21) Application number: 99119918.3
(22) Date of filing: 08.10.1999
(51) Int. Cl.: A61B 10/00

(54) **Biopsy device**

(30) Priority: 09.10.1998 IT BO980569
(71) Applicant: Gallini S.R.L, 41037 Mirandola (IT)
(72) Inventor: Avaltroni, Paolo, 46100 Mantova (IT)
(74) Representative: Dall'Olio, Giancarlo

(57) **Abstract**

The needle device (S) for bone biopsy includes a cylindrical hollow cannula (1). The device (S) includes also a stylet (3), whose distal end (3b) is pointed and which is to be introduced slidingly into the cannula (1,101). The length of the stylet (3) is such that the pointed distal end (3b) goes out from the distal end (1b) of the cannula (1), when the stylet is wholly introduced therein. The device (S) includes at least one blade (43), which is situated at the distal end of a needle (4) and which extends longitudinally and centrally inside the cannula (1), when the above mentioned needle (4) is wholly introduced into the cannula (1), so as to extend along at least a distal portion of the latter, which is aimed at receiving a bioptic sample (6).

## Description

The present invention relates to the production of surgical instruments for sampling tissue of humans and animals, usually for diagnosis.

In particular, the invention relates to a needle device for sampling either osteo-medullary or soft tissues.

Different types of devices for biopsy, i.e. for removing small parts of either rigid, like bone or osteo-medullary tissue, or soft tissues have been known for a long time.

In particular, rigid tissues, as e.g. bony and/or osteo-medullary tissues, are traditionally sampled by a cannula, that has a handle made at its proximal end while the other, distal end is tapered and usually has a sharp edge.

A stylet, which is a cylindrical needle, is slidably inserted into the cannula. The stylet has also a handle made at its proximal end while its other, distal end is suitably shaped.

The length of the stylet is such that the sharp end protrudes a bit from the cannula, when the stylet is completely inserted therein.

The above described device is manually pressed to be introduced through the skin and possible adipose tissue and muscular bundle up to the rigid tissue to be removed.

This stylet facilitates the tissue penetration and perforation, even the most rigid outer part of bone tissue, and prevents tissue other than the one to be sampled from introducing into the cannula.

After the stylet has perforated the tissues, it is withdrawn from the cannula and the cannula is pushed forward with a repeated rotary movement, so as to cut a cylindrical portion of tissue, technically known as "frustule", and to pull it thereinside.

Then, the cannula is further rotated and oscillated in order to detach, hopefully, the above mentioned cylindrical portion from the surrounding tissue, so as to separate the sample to be examined.

Finally, the cannula containing the cylindrical bioptic sample is withdrawn from the tissue.

The most frequent disadvantages of these devices derive from the fact that the operations are invasive as well as from the ways, in which the tissue is removed.

In fact, it is very probable that, after the detaching movements, the bioptic sample is not wholly separated from the surrounding tissue, and therefore, it is not removed together with the cannula, thus rendering the biopsy unsuccessful.

Moreover, the cannula is imparted strong rotary and oscillating movements that are necessary for resection of the sample from the surrounding tissue, but that cause further shock and micro-fractures of the bony and/or osteo-medullary tissues, thus increasing patient's pains and slowing down the recovery.

One of the devices which try to overcome, at least partially, these disadvantage, is described in the Italian Patent No. 1.261.099. According to this document, after the stylet has been removed, a cylinder is introduced into the cannula. The cylinder, whose outer diameter is not bigger than the inner diameter of the cannula, is provided, at its distal end, with a thin blade, of approximately semi-circular shape, which partially continues the shape of the cylinder.

The cylinder is introduced into the cannula until the blade reaches a position between the inner wall of the cannula and the sample of tissue, pressing the latter also due to the tapering of the distal end of the cannula.

Although using the above described device the frustule adheres much more to the inner wall of the cannula, the device maintains the continuity between the distal end of the frustule and the surrounding tissue.

Thus, this distal end of the frustule must be detached by means of operations which provoke its tearing.

The bony biopsy technique has been further improved by the device disclosed in the Italian Patent Application No. BO96A 000271 of the same Applicant.

This last biopsy device includes a hollow cylinder which is introduced into the cannula, after the stylet has been withdrawn.

At its distal end, the hollow cylinder is provided with resection means, which protrude from the distal end of the cannula when the cylinder is wholly introduced therein.

The resection means include at least a pair of opposite lobes in form of cylindrical portions, that continue the shape of the hollow cylinder up to a predetermined length.

The lobes cut the terminal part of the bioptic sample and keep it.

The resection of the bioptic sample is facilitated by the converging tapering of the distal portion of the cannula, which guides the above mentioned lobes until they substantially join.

In this way, most of the frustule end is cut off, and the frustule is kept inside the joined lobes.

Thus, the patient's suffering is reduced and the frustule is bigger, with the same usual needle penetration.

The main object of the present invention is to propose a device for bone biopsy, which can be used also for soft tissue biopsy and which guarantees that the bioptic sample is completely detached from the surrounding tissue and removed.

Another object of the present invention is to provide a biopsy device obtained by a simple, cheap, extremely functional and reliable technical solution, which makes the above described operations simple, rapid and efficient.

The above mentioned objects are obtained in accordance with the invention as disclosed in claim 1.

The characteristics of the invention have been pointed out in the following, with a particular reference to the enclosed drawings, in which:
- Fig. 1 shows a schematic longitudinal sectional view of a first embodiment of the proposed biopsy device, with the stylet introduced;
- Figure 2 shows a schematic, enlarged, sectional, side view of a portion A of the device of Figure 1;
- Figure 3 shows a schematic, sectional, side view of a first embodiment of the proposed biopsy device provided with frustule resection means;
- Figure 4 shows a schematic, enlarged, sectional, side view of a portion B of the device of Figure 3:
- Figures from 5a to 5e show schematic and enlarged views of a working sequence of a biopsy carried out by the device shown in previous Figures;
- Figure 6 shows a schematic, enlarged, sectional, side view of the distal portion of the proposed device according to a second embodiment of the resection means;
- Figure 7 shows a schematic, enlarged, sectional, side view of the distal portion of the proposed device according to a third embodiment;
- Figure 8 shows the corresponding stylet which can be used with the device of Figure 7.

With reference to the above described figures, a biopsy device is indicated with S.

Although it has been evolved mainly for biopsy of rigid tissue, like bone and/or osteo-medullary tissues, its peculiar and original conformation allows it to be used also for soft tissues biopsy.

The following description of the device S refers to osteo-medullary tissue biopsy, but it is understood that the same working technique can be used for biopsy of soft, not too yielding, tissues.

According to a first embodiment, the device S includes a metallic, substantially cylindrical cannula 1, which is open at both ends and equipped, in the region of its proximal end 1a, with a handle 11, whose shape facilitates its handling and use.

The distal end 1b of the above mentioned cannula 1 is serrated and sharp, so that the cannula easily penetrates inside the tissue, from which a bioptic sample 6 is to be withdrawn.

The handle 11 not only facilitates manual handling, but it is also shaped in such a way as to leave the proximal end 1a of the cannula 1 free and accessible from outside.

A substantially cylindrical stylet 3, made preferably of metallic material, is introduced slidingly into the cannula 1 through its proximal end 1a.

At its proximal end 3a, the stylet 3 includes a handle 31, whose shape matches complementary with the handle 11 of the cannula 1, when the stylet 3 has been wholly introduced into the cannula.

The distal end 3b of the stylet 3 is pointed and goes out from the distal end 1b of the cannula 1, when the stylet has been wholly introduced therein, until the respective handles 31, 11 match with each other.

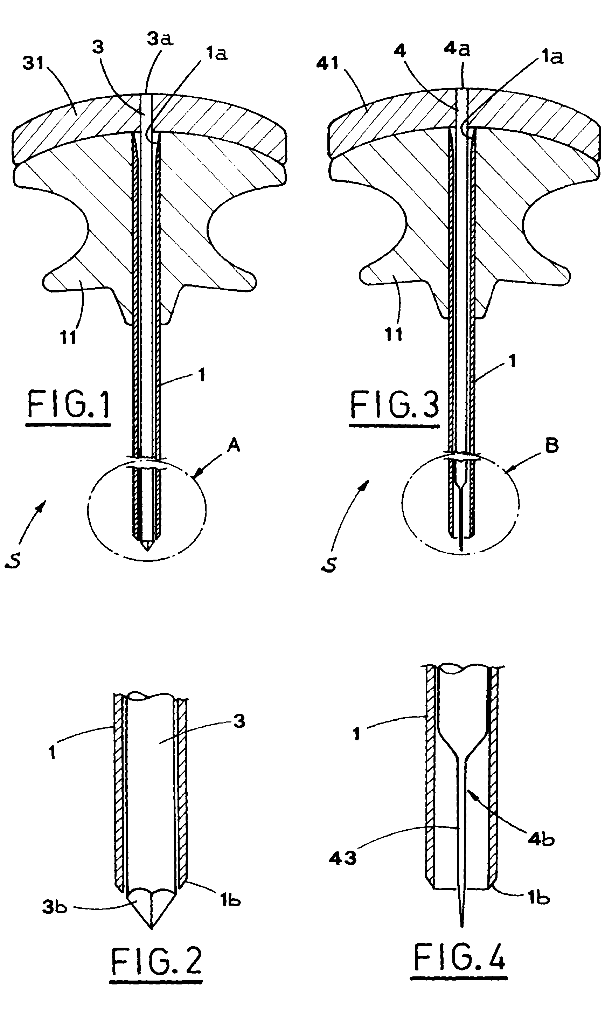

The device S includes also a cylindrical needle 4 (see Figures 3 and 4), made of suitable metallic material and aimed at being introduced inside the cannula 1, when the stylet 3 has been withdrawn therefrom.

The proximal end 4a of the cylindrical needle 4 has a handle 41, whose shape matches complementary with the handle 11 of the cannula 1, when the cylindrical needle 4 has been wholly introduced into the cannula.

At its distal end 4b, the needle 4 extends as a substantially rectangular blade 43, whose end 43a is particularly sharp and preferably forms a very narrow wedge.

It is preferable that the blade 43 extends along the diametrical plane of the needle 4, although this is not necessary, since the blade 43 arrangement on a non diametrical plane would be equally advantageous.

Preferably, but not necessarily, the length of the needle 4 is such as to make a minimum portion of the blade 43 go out from the distal end 1b of the cannula 1, when the needle is wholly introduced into the cannula 1, until the handle 11 of the cannula 1 match with the handle 41 of the needle.

When the biopsy is to be carried out, the surgeon handles the device S with the stylet 3 already in operative position, and introduces the device through a surface layer M of adipose tissue and muscular bundle up to a position close to a bone tissue from which the bioptic sample 6 is to be removed.

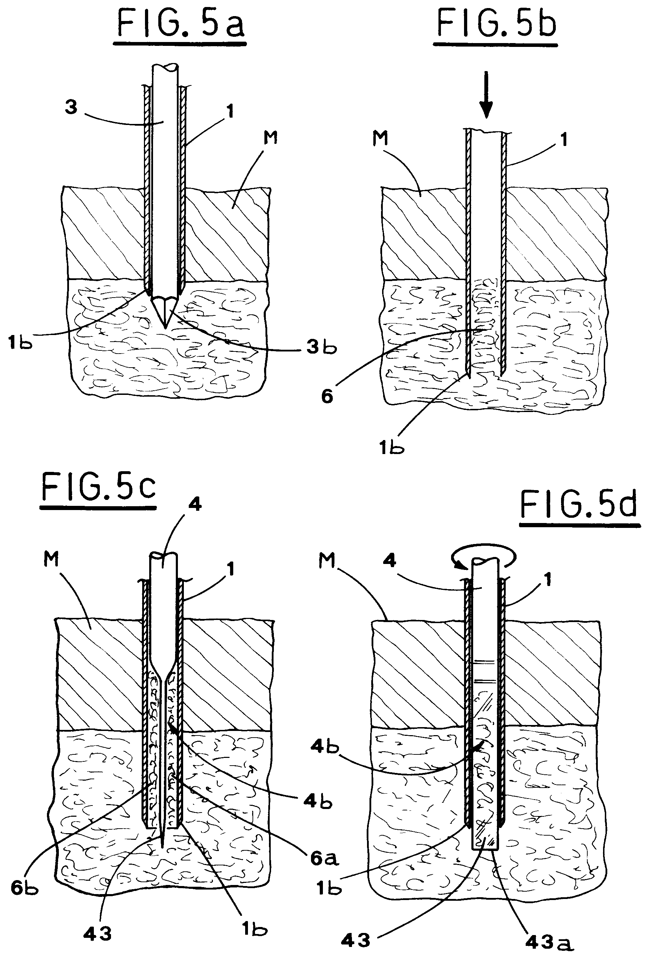

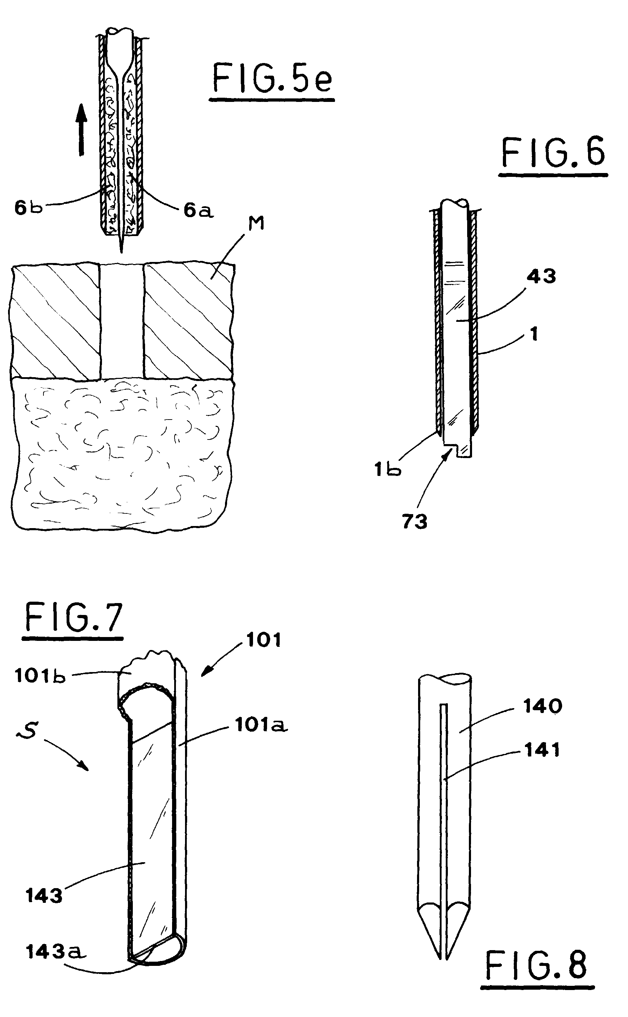

Afterwards, the surgeon withdraws the stylet 3 from the cannula 1, pushes the latter inside the bone or osteo-medullary tissue (Figure 5b), until a substantially cylindrical portion of the tissue, forming the bioptic sample 6, is contained in the cannula 1.

At this point, the surgeon introduces wholly the needle 4 inside the cannula 1 (Figure 5c), until the handle 41 of the needle adheres to the handle 11 of the cannula.

Thus, the blade 43 penetrates inside the bioptic sample 6 dividing it in two portions 6a and 6b, and partially going out from the distal end 1b of the cannula 1.

In this configuration of the device S, the blade 43, also due to its wedge shape, slightly pushes the two portions 6a and 6b, so as to press them against the walls of the cannula 1.

At this point, the surgeon rotates the device by 180° or a bit more (Figure 5d).

Thus the group including the cannula 1 and the blade 43 makes also the bioptic sample 6 to rotate, whose end is separated from the surrounding tissue by the cutting action of the blade 43.

Afterwards, the surgeon withdraws the device S containing, between the wall of the cannula 1 and the wall of the blade 43, the two portions of the bioptic sample 6 to be examined.

According to a use variant of the device S, the needle 4 is introduced into the cannula 1 immediately after the stylet 3 has been withdrawn, before the sample 6 has been removed. This use of this variant is completely within the surgeon's discretion.

According to a second embodiment of the device S, shown in Figure 6, the distal portion of the blade 43, which goes out from the cannula 1, has a recess 73, whose extension is equal or smaller than half of the blade 43 width.

The use of the device S obtained according to the above described second embodiment, is identical with the use of the device according to the first embodiment, described before.

In particular, the distal end of the bioptic sample 6 is likewise cut off during a 180 degree rotation of the device S.

Thus, the production of the needle 4 is simpler and cheaper, because the extension of the blade 43 end to be sharpened accurately is much smaller.

According to a third embodiment, shown in Figures 7 and 8, the blade, indicated in this case with 143, is made directly in the distal portion of the relative cannula 101, and its length is at least equal with the size of the bioptic sample 6 to be removed.

Also according to this embodiment, it is preferable, but not wholly necessary, that the blade 143 occupies diametrically the inside of the cannula 101.

The so shaped cannula 101 can be easily obtained by known consolidated production techniques. For instance, it is possible to obtain the cannula 101 by using two semi-cylindrical shells 101a, 101b. The blade 143 is fastened inside one of the shells 101a, 101b, which are welded, so as to form the cannula 101 containing the blade 143.

Obviously, a corresponding stylet 140, which is used with the above described cannula 101, has a longitudinal groove 141 for receiving the blade 143 when the stylet 140 is introduced into the cannula 101.

According to the above described embodiment, the device S introduction up to the removal area is identical with the other previously described embodiment.

Afterwards, the stylet 140 is withdrawn, and the cannula 101 with the blade 143 is introduced into the tissue to be removed for a predetermined length.

The so obtained bioptic sample 6 is already separated longitudinally in two portions and is definitely separated from the surrounding tissue by subsequent 180 degree rotation of the device S, as in previously described cases.

It has appeared that the device S obtained according to the last embodiment, is particularly suitable for biopsy of not only for osteo-medullary tissue, but also soft tissue.

Moreover, the use of the so obtained device S is simpler because it includes one moving element less than the device obtained according to previous embodiments, yet maintaining the complete resection effect obtained by the blade 143 rotation.

The device S according to all previously described embodiments, with the usual cannula diameter and penetration depth, advantageously allows to remove a bioptic sample, which is much bigger with respect to samples removed by the device used so far.

This is possible because the distal portion of the cannula 1, 101 does not have to be necessarily tapered, which is extremely important for the device invasiveness, since it is possible to use a cannula of smaller diameter to remove a sample of required dimensions.

Moreover, due to advantageous conformation of the resection means, the above described device reduces patient's suffering.

It is also to be pointed out that the elements forming the biopsy device are simple to obtain, extremely strong and efficient and that their number is limited. All this leads to reduction of production costs.

Obviously, there are other possible variants, which are different from the above described and illustrated embodiments and which remain within the scope of the present invention.

For instance, according to a fourth embodiment, the blade 43, 143 is not flat, but helical.

This leads to other advantages of separation and withdrawal of the sample 6, because the conformation of the blade, together with the device rotation in a suitable direction, tends to create forces which push the sample toward the inside of the cannula.

## Claims

1. Needle device for bone biopsy, including a hollow cannula (1,101), a stylet (3,140), whose distal end (3b) is pointed and which is to be introduced into said cannula (1,101), the length of said stylet (3,140) being such that the pointed distal end (3b) goes out from the distal end (1b) of the cannula (1,101), when the stylet is wholly introduced therein, said device (S) being characterized in that it includes at least one blade (43,143), which extends longitudinally inside said cannula (1,101), near a diametrical plan thereof, along at least a distal portion of said cannula which is aimed at receiving a bioptic sample (6), with said blade (43,143) being aimed at separating said bioptic sample (6) from the surrounding tissue by its distal end (43a, 143a).

2. Device, according to claim 1, characterized in that said blade (43) is joined to the distal end (4b) of a needle (4), which is to be introduced into said cannula (1) after said stylet (3) has been removed therefrom.

3. Device, according to claim 2, characterized in that a minimum portion of said blade (43) goes out of the distal end of said cannula (1).

4. Device, according to claim 2, characterized in that at its distal end (43a), said blade (43) features a recess (73) extending from an outer edge of said blade (43) toward the inside thereof, by a length which is equal or smaller than half of said blade (43) width.

5. Device, according to claim 2, characterized in that said needle (4) is equipped, at its proximal end (4a), with a handle (41), whose shape is such as to perfectly match the handle (11) of the cannula (1), when said needle (4) is wholly introduced into said cannula.

6. Device, according to any of claims from 2 to 4, characterized in that the thickness of said blade (43) is decreasing toward its distal end (43a).

7. Device, according to claim 1, characterized in that said blade (143) is fastened to said cannula (101) and in that said stylet (140) features a longitudinal groove (141), which is at least as deep as the length of said blade (143), said longitudinal groove (141) being aimed at receiving said blade (143) when the stylet (140) is introduced into said cannula (101).

8. Device, according to claim 1, characterized in that said blade (43,143) extends longitudinally inside said cannula (1), and is substantially flat.

9. Device, according to claim 8, characterized in that said blade (43,143) lies on a diametrical plane of said cannula (1).

10. Device, according to claim 1, characterized in that said blade has a helical shape.
